# EUROPEAN PATENT APPLICATION

(11) **EP 4 768 466 A1**
(43) Date of publication of application: **01.07.2026**
(21) Application number: 24856685.3
(22) Date of filing: 30.07.2024
(51) Int. Cl.: C07C 69/84, C07C 67/52, C08J 11/24, B01D 15/36, C08G 63/06, C08G 63/60

(54) **RECYCLED BHET HAVING LOW YELLOW INDEX AND MANUFACTURING METHOD OF POLYESTER RESIN**

(30) Priority: 22.08.2023 KR 20230110158
(71) Applicant: SK Chemicals Co., Ltd., Seongnam-si, Gyeonggi-do 13494 (KR)
(72) Inventor: KIM, Ji-Hun, Seongnam-si, Gyeonggi-do 13494 (KR); PARK, Jun-Yong, Seongnam-si, Gyeonggi-do 13494 (KR); RYU, Gayeong, Seongnam-si, Gyeonggi-do 13494 (KR); LEE, Joong Ki, Seongnam-si, Gyeonggi-do 13494 (KR)
(74) Representative: Haseltine Lake Kempner LLP
(86) International application number: PCT/KR2024/011167
(87) International publication number: WO 2025/042072

(57) **Abstract**

This method comprising the steps of reacting waste polyester with a chromophore decomposing agent after depolymerization and separating and removing bis(2-hydroxyethyl)terephthalate can provide regenerated bis(2-hydroxyethyl)terephthalate having a low yellow index from highly contaminated waste polyester

## Description

### Technical Field

The present invention relates to a process for preparing recycled BHET with a low yellow index and to a process for preparing a polyester resin using the same.

### Background Art

Polyester is widely used as a material for beverage-filling containers, packaging films, audio and video films, and the like by virtue of its excellent mechanical strength, thermal resistance, transparency, and gas barrier properties. In addition, polyester is widely produced worldwide as an industrial material such as medical fibers and tire cords. In particular, polyester sheets or plates have good transparency and excellent mechanical strength, so that they are widely used as raw materials for cases, boxes, partitions, shelves, panels, packaging materials, building materials, interior and exterior materials, and the like.

As a result, waste of plastics such as polyester is generated globally at an unmanageable level every year. Recently, countries around the world have prepared regulations and plans for recycling waste plastic resources, including waste polyester. Although physical or chemical methods are used as methods of recycling waste polyester, physical recycling methods cannot guarantee purity and, thus, are not widely used.

Meanwhile, in chemical recycling methods, the ester bond of waste polyester is severed to depolymerize it. Specifically, reactions such as glycolysis, hydrolysis, methanolysis, and aminolysis are used. Glycolysis among them is to decompose waste polyester by adding a glycol such as ethylene glycol or diethylene glycol at high temperatures. A reaction product comprising mainly bis(2-hydroxyethyl) terephthalate (BHET) is obtained. The bis(2-hydroxyethyl) terephthalate contained in the reaction product may be used as a raw material for preparing unsaturated polyester or ester polyol after the crystallization or purification thereof.

However, bis(2-hydroxyethyl) terephthalate recycled in this way may contain reagents or solvents used in various chemical steps during the depolymerization of waste polyester, or by-products formed by side reactions with them. These impurities may remain in trace amounts even after several rounds of purification. In particular, the additives used in the initial production of a polyester are rich in substances with a molecular structure that has strong coloring. In addition, these substances with a coloring structure may be formed or incorporated after the preparation of a polyester. Bis(2-hydroxyethyl) terephthalate recovered from such contaminated waste polyester has low color quality and can be hardly used in the production of a polyester resin with high quality.

### [Prior Art Document]

(Patent Document 1) Korean Laid-open Patent Publication No. 2022-0068991

### Disclosure of Invention

### Technical Problem

If recycled bis(2-hydroxyethyl) terephthalate has poor color quality, it has a direct impact on the color of a polyester resin prepared using it. However, it was not easy to improve the color of such recycled bis(2-hydroxyethyl) terephthalate using conventional techniques.

As a result of research conducted by the present inventors, the yellow index of recycled bis(2-hydroxyethyl) terephthalate could be significantly lowered through a process of depolymerizing highly contaminated waste polyester, reacting it with a chromophore decomposing agent, and separating and removing the chromophore decomposing agent from BHET.

Accordingly, an object of the present invention is to prepare recycled bis(2-hydroxyethyl) terephthalate with a low yellow index from contaminated waste polyester and prepare a recycled polyester resin with a high white index using it.

### Solution to Problem

According to an aspect of the present invention, there is provided a process for preparing recycled bis(2-hydroxyethyl) terephthalate, which comprises (1) obtaining a depolymerization product by glycolysis of waste polyester; (2) reacting the depolymerization product with a chromophore decomposing agent to obtain a decomposition product in which a chromophore is decomposed; (3) adsorbing and removing the chromophore in the decomposition product with an ion-exchange resin to obtain a purified product; and (4) crystallizing the purified product.

According to another aspect of the present invention, there is provided recycled bis(2-hydroxyethyl) terephthalate, which is prepared by the above process, wherein the yellow index (YI) is 7 or less when measured for a solution dissolved in ethylene glycol at a concentration of 50% by weight.

According to another aspect of the present invention, there is provided a process for preparing a polyester resin, which comprises (1) obtaining a depolymerization product by glycolysis of waste polyester; (2) reacting the depolymerization product with a chromophore decomposing agent to obtain a decomposition product in which a chromophore is decomposed; (3) adsorbing and removing the chromophore in the decomposition product with an ion-exchange resin to obtain a purified product; (4) crystallizing the purified product to obtain recycled bis(2-hydroxyethyl) terephthalate; and (5) polymerizing a polyester resin using the recycled bis(2-hydroxyethyl) terephthalate.

According to another aspect of the present invention, there is provided a polyester resin prepared by the above process.

### Advantageous Effects of Invention

According to the process of the present invention, once waste polyester has been depolymerized, it is reacted with a chromophore decomposing agent, and the resulting decomposed chromophore and by-products are separated and removed. As a result, even if the contamination level of waste polyester is high, the yellow index of bis(2-hydroxyethyl) terephthalate recovered from it can be significantly lowered, and the content of impurities can be reduced to a minimum level. Accordingly, a polyester resin prepared using recycled bis(2-hydroxyethyl) terephthalate obtained by the above process has a high white index and excellent quality.

### Brief Description of Drawings

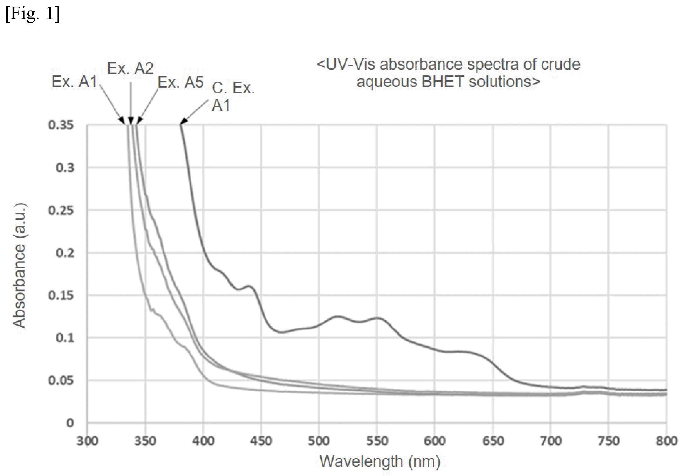

Fig. 1 shows the UV-Vis absorbance spectra of crude aqueous solutions of bis(2-hydroxyethyl) terephthalate according to an embodiment of the present invention.

### Best Mode for Carrying out the Invention

In this specification, terms referring to the respective components are used to distinguish them from each other and are not intended to limit the scope of the embodiment. In addition, in the present specification, a singular expression is interpreted to cover a plural number as well unless otherwise specified in the context.

In the present specification, the terms first, second, and the like are used to describe various components. But the components should not be limited by the terms. The terms are used for the purpose of distinguishing one element from another.

In the present specification, the term "comprising" is intended to specify a particular characteristic, region, step, process, element, and/or component. It does not exclude the presence or addition of any other characteristic, region, step, process, element, and/or component, unless specifically stated to the contrary.

The molecular weight of a compound or polymer described in the present specification, for example, a number average molecular weight or a weight average molecular weight, is a relative mass based on carbon-12 as is well known. Although its unit is not described, it may be understood as a molar mass (g/mole) of the same numerical value, if necessary.

In the numerical range that limits the size and physical properties of components and the like described in the present specification, when a numerical range limited with the upper limit only and a numerical range limited with the lower limit only are separately exemplified, it should be understood that a numerical range combining these upper and lower limits is also encompassed in the exemplary scope of the invention.

According to an aspect of the present invention, there is provided a process for preparing bis(2-hydroxyethyl) terephthalate with a low yellow index in which waste polyester is depolymerized, and a chromophore is decomposed and removed.

The process for preparing recycled bis(2-hydroxyethyl) terephthalate according to an embodiment of the present invention comprises (1) obtaining a depolymerization product by glycolysis of waste polyester; (2) reacting the depolymerization product with a chromophore decomposing agent to obtain a decomposition product in which a chromophore is decomposed; (3) adsorbing and removing the chromophore in the decomposition product with an ion-exchange resin to obtain a purified product; and (4) crystallizing the purified product.

In addition, the process may further comprise, before step (2), distilling moisture and a residual solvent in the depolymerization product.

In addition, the process may further comprise, before the crystallization in step (4), adding an adsorbent to the purified product and carrying out adsorption.

According to the process of the present invention, once waste polyester has been depolymerized, it is reacted with a chromophore decomposing agent, and the resulting decomposed chromophore and by-products are separated and removed. As a result, even if the contamination level of waste polyester is high, the yellow index of bis(2-hydroxyethyl) terephthalate recovered from it can be significantly lowered, and the content of impurities can be reduced to a minimum level. Accordingly, a polyester resin prepared using recycled bis(2-hydroxyethyl) terephthalate obtained by the above process has a high white index and excellent quality.

Hereinafter, the recycled bis(2-hydroxyethyl) terephthalate and the process for preparing the same will be described in detail.

### Depolymerization of waste polyester

First, a depolymerization product is obtained by glycolysis of waste polyester.

The waste polyester may be obtained from a polyester material product discarded after use. Specifically, the waste polyester may comprise waste products such as beverage bottles, fabrics, films, cases, boxes, partitions, shelves, protective panels, packaging materials, building materials, and interior and exterior materials, which comprise various polyester materials (e.g., polyethylene terephthalate (PET) material) discarded after having been used by consumers.

The waste polyester may be contaminated waste polyester. The contaminants present in the waste polyester vary considerably. The contaminants comprise, for example, additives used in the initial production of a polyester and substances formed during use or entrained by contamination. In particular, the additives used in the initial production of a polyester are rich in substances with a molecular structure that has strong coloring. These substances with a coloring structure may be formed or incorporated after the preparation of a polyester.

For example, the waste polyester may have a yellow index (YI), when measured in a solid phase, of 5 or more, 6 or more, 7 or more, 8 or more, 9 or more, 10 or more, 15 or more, or 20 or more, as a specific example, 5 to 100 or 10 to 100. In an embodiment, the yellow index (YI) of the waste polyester when measured in a solid phase may be 10 or more.

The waste polyester may be pretreated before being subjected to depolymerization. The pretreatment may be carried out by removing other plastics, metals, and foreign substances mixed in the waste, washing it, and then crushing it through a crusher. As a result of the pretreatment, the waste polyester may have a flake form. In addition, the waste polyester may have a fine structure like a fiber.

The waste polyester pretreated in this way is then subjected to a depolymerization process. The depolymerization process may comprise, for example, a glycolysis reaction. As is well known, the glycolysis reaction refers to a chemical reaction in which a polymer chain or the like is severed by a glycol such as ethylene glycol. The total weight of the glycol added may be 1, 2, or 3 times the weight of the waste polyester resin or more and may be 7, 5, or 4 times or less. For example, the weight of the glycol added may be 1 to 7 times, specifically, 2 to 5 times, more specifically, 3 to 4 times, relative to the weight of the waste polyester resin.

A catalyst may be used in the glycolysis reaction. The catalyst may be a metal catalyst, for example, a metal salt catalyst or a metallic organic catalyst. Specifically, the catalyst may be an acetate, carbonate, oxide, or hydroxide of a metal, and the metal may be an alkaline metal, an alkaline earth metal, or a transition metal. As a specific example, the catalyst comprises a metal acetate, or an anhydride or a hydride thereof. More specifically, it may be at least one selected from the group consisting of zinc acetate, sodium acetate, cobalt acetate, and manganese acetate, or in the form of a hydrate or anhydride thereof. In addition, the weight of the catalyst added may be 0.01 part by weight or more, 0.1 part by weight or more, 0.2 part by weight or more, or 0.3 part by weight or more, and may be 5 parts by weight or less, 1 part by weight or less, 0.7 part by weight or less, 0.5 part by weight or less, or 0.4 part by weight or less, relative to 100 parts by weight of the waste polyester resin. For example, the weight of the catalyst added may be 0.1 part by weight to 1 part by weight, specifically, 0.2 part by weight to 0.7 part by weight, relative to 100 parts by weight of the waste polyester resin. More specifically, the catalyst may be used in an amount of 0.2 part by weight to 0.4 part by weight relative to 100 parts by weight of the waste polyester.

As a specific example, the glycolysis in step (a) may comprise a reaction of waste polyester and ethylene glycol in the presence of an acetate-based catalyst.

The depolymerization may comprise, for example, a multi-stage depolymerization reaction at low temperatures. According to an embodiment, the depolymerization comprises subjecting waste polyester to depolymerization through a first glycolysis reaction at a high temperature; and subjecting the product to depolymerization at a low temperature through a second glycolysis reaction.

The temperature during the first glycolysis reaction may be 170°C or higher, 180°C or higher, or 190°C or higher, and may be 220°C or lower, 210°C or lower, 200°C or lower, 195°C or lower, or 190°C or lower. For example, the temperature during the first glycolysis reaction may be 180°C to 210°C, specifically, 180°C to 200°C, more specifically, 180°C to 190°C.

In addition, the temperature during the second glycolysis reaction may be 140°C or higher, 150°C or higher, or 160°C or higher, and may be 180°C or lower, 170°C or lower, or 160°C or lower. For example, the temperature during the second glycolysis reaction may be 150°C to 170°C, specifically, 150°C to 160°C, more specifically, 150°C to 155°C.

As a specific example, the step of obtaining the depolymerization product may comprise (1) obtaining a first depolymerization product by a first glycolysis reaction of waste polyester at a temperature of 180 to 210°C; and (2) obtaining a second depolymerization product by a second glycolysis reaction of the first depolymerization product at a temperature of 150 to 170°C.

The period of time required for the first and second glycolysis reactions may be 1 hour or more or 2 hours or more, and may be 4 hours or less or 3 hours or less, from the point when the appropriate temperature is reached. For example, the period of time required for the first and second glycolysis reactions may be 1 hour to 4 hours, specifically, 1 hour to 3 hours, more specifically, 1 hour to 2 hours, from the point when the appropriate temperature is reached.

As a more specific example, the first glycolysis reaction may be carried out at a temperature of 180°C to 190°C for 1 hour to 3 hours. In addition, the second glycolysis reaction may be carried out at a temperature of 150°C to 160°C for 1 hour to 3 hours.

As an example, the first glycolysis reaction may be carried out in the presence of a zinc acetate anhydride catalyst. As a specific example, the first glycolysis reaction may be carried out at a temperature of 180°C to 200°C for 1 hour to 3 hours in the presence of a zinc acetate anhydride catalyst. The zinc acetate anhydride may be used in an amount of 0.2 part by weight to 0.4 part by weight relative to 100 parts by weight of the waste polyester. In addition, the second glycolysis reaction may be carried out at a temperature of 140°C to 160°C for 1 hour to 3 hours upon the further addition of ethylene glycol without an additional catalyst.

### Subsequent process to depolymerization

The depolymerization product (crude bis(2-hydroxyethyl) terephthalate solution) obtained through glycolysis may be subjected to a subsequent process such as cooling, filtration, adsorption, distillation, and solvent recovery.

In an embodiment, the depolymerization product may be subjected to cooling. The cooling temperature may be, for example, 150°C or lower, 140°C or lower, 130°C or lower, 120°C or lower, 110°C or lower, 100°C or lower, and may be 50°C or higher, 60°C or higher, 70°C or higher, 80°C or higher, or 90°C or higher.

Thereafter, insoluble foreign substances may be removed from the cooled depolymerization product through filtration. As a specific example, a step of cooling the depolymerization product to 120°C or lower and filtering it upon the addition of a filter aid may be further carried out. As a result, fine particles and insoluble organic substances present in the depolymerization product can be filtered out by solid-liquid separation.

Since bis(2-hydroxyethyl) terephthalate (BHET) or oligomers obtained through the depolymerization reaction are present in a solid form at room temperature, it is difficult to separate foreign substances at room temperature. Thus, it is preferable to separate them at a temperature condition of 90°C to 150°C, more specifically, 110°C to 150°C. In addition, if the above temperature range is maintained, the removal of insoluble foreign substances may be facilitated thanks to good flowability.

Various methods and devices may be used in the removal of insoluble foreign substances through solid-liquid separation. For example, a device such as a pressurized filter, a centrifugal separator, a filter press, a belt press, or the like may be used. But it is not limited thereto as long as any method capable of separating foreign substances is used.

In addition, the depolymerization product may be further subjected to ion exchange through an ion-exchange resin. As it is subjected to the ion exchange, ionic impurities present in the depolymerization product, specifically, catalysts and metallic foreign substances, may be removed.

In addition, the depolymerization product may be further subjected to removing moisture and a residual solvent by distillation.

Since an unreacted glycol still remains in the depolymerization product after filtration thereof in the previous step, it is necessary to remove the same from the product prior to the next step.

In addition, it is necessary to perform a step of recovering an unreacted glycol for an economical depolymerization process. That is, it is possible to recover and reuse a glycol, among glycols such as ethylene glycol, propylene glycol, diethylene glycol, or the like previously employed in the depolymerization, that remains without participating in the glycolysis reaction.

The distillation to remove the unreacted glycol may be carried out by, for example, vacuum distillation. A glass distillation apparatus or a rotary evaporator may be used for this purpose.

As the vacuum distillation to remove the unreacted glycol is carried out at a temperature of 150°C or lower, the purity of BHET can be enhanced by further reducing the formation of diethylene glycol and impurities derived therefrom. For example, the vacuum distillation to remove the unreacted glycol may be carried out at a temperature of 150°C lower, 130°C lower, or 120°C lower, and 80°C higher, 90°C higher, 100°C higher, or 110°C higher. Specifically, the temperature during the distillation to remove the unreacted glycol may be 80°C to 190°C or 90°C to 150°C. As a more specific example, the distillation to remove the unreacted glycol may be carried out at a temperature of 100°C to 130°C.

The pressure during the vacuum distillation to remove the unreacted glycol may be, for example, 0.1 Torr to 760 Torr, 0.1 Torr to 200 Torr, or 0.5 Torr to 30 Torr. More specifically, the vacuum distillation may be carried out under stepwise reduced pressure conditions from 760 Torr to 0.8 Torr.

In addition, the depolymerization product may be prepared through a further step of thin film distillation under a reduced pressure.

The thin-film distillation is a distillation method that makes a mixture to be separated into a thin film for increasing its surface area in contact with a heat source. Specifically, a mixture fed to the evaporator of the thin film evaporator forms a thin film on the inner wall of the thin film evaporator by the wiper rotor. Then, distillation is carried out under appropriate temperature conditions by heating. In addition, a condenser for recovering the evaporated material may be provided inside the thin film evaporator.

The thin film evaporation may be carried out by short path evaporation. Since such a short path and thin film evaporation has a short residence time and enables vacuum distillation using a high vacuum, it is possible to separate high-boiling or high-molecular-weight materials that are hardly separated by other distillation methods while minimizing the change of the reactants by heat. In addition, if the pressure inside a thin film evaporator is lowered, there is an advantage in that the vapor pressure of a material is reduced, which allows evaporation to take place at a lower temperature than its original boiling point.

As a specific example, the depolymerization product is fed to a short path and thin film evaporator, and a wiper for forming a thin film is rotated at 300 rpm or more. As a result, a vaporized material and a non-vaporized material can be separated from each other. The internal thin film temperature of the upper thin film evaporation apparatus during the thin film evaporation may be, for example, 150°C to 250°C, 190°C to 250°C, or 180°C to 220°C. In addition, the internal pressure of the upper thin film evaporation apparatus during the thin film evaporation may be, for example, 0.005 Torr to 5.0 Torr, 0.05 Torr to 5.0 Torr, 0.05 Torr to 1.5 Torr, or 0.05 Torr to 1 Torr.

### Decomposition of a chromophore

Thereafter, the depolymerization product is reacted with a chromophore decomposing agent to obtain a decomposition product in which a chromophore is decomposed.

For example, the chromophore decomposing agent may comprise an oxidizing agent. Specifically, it may comprise at least one selected from the group consisting of a chlorine-based oxidizing agent, an oxygen-based oxidizing agent, and hydrogen peroxide. Examples of the chlorine-based oxidizing agent include chloramines, chlorates, chloric acids, chloroisocyanuric acids, and chlorine dioxide. Examples of the oxygen-based oxidizing agent include hydrogen persulfate, persulfate, percarbonate, and perborate.

Specific examples of the chloroamines include chloramine T (N-chloro-P-toluenesulfonamide sodium), dichloramine T (N,N'-dichloro-P-toluenesulfonamide), chloramine B (N-chlorobenzenesulfonamide sodium), and dichloramine B (N,N'-dichlorobenzenesulfonamide).

Specific examples of the chlorates include sodium chlorate, potassium chlorate, lithium chlorate, ammonium chlorate, calcium chlorate, sodium hypochlorite, potassium hypochlorite, calcium hypochlorite, sodium chlorite, disodium chlorite, potassium perchlorate, rubidium perchlorate, cesium perchlorate, ammonium perchlorate, potassium perchlorate, calcium perchlorate, silver perchlorate, sodium perchlorate, and magnesium perchlorate.

Specific examples of the chloric acids include perchloric acid (HClO₄), chloric acid (HClO₃), and hypochlorous acid (HClO).

Specific examples of the chloroisocyanuric acids include chloroisocyanuric acid, sodium chloroisocyanurate, potassium chloroisocyanurate, dichloroisocyanuric acid, sodium dichloroisocyanurate, potassium dichloroisocyanurate, trichloroisocyanuric acid, sodium trichloroisocyanurate, and potassium trichloroisocyanurate.

In the decomposition reaction, one type of chromophore decomposing agent may be used alone, or two or more types of chromophore decomposing agents may be used in combination. When two or more types of chromophore decomposing agents are used, they may be added to the depolymerization simultaneously or sequentially.

In an embodiment, the chromophore decomposing agent may comprise a chlorine-based oxidizing agent.

As a specific example, the chromophore decomposing agent may comprise chloroamines. As a more specific example, the chromophore decomposing agent may comprise Chloramine-T, which may be more effective in producing the chromophore decomposition effect than other chloramines.

As another specific example, the chromophore decomposing agent may further comprise at least one of chlorates, chloric acids, and hydrogen peroxide, in addition to chloroamines. As a more specific example, the chromophore decomposing agent may further comprise at least one selected from the group consisting of NaClO, NaClO₄, HClO₄, HClO, and H₂O₂, in addition to chloroamines. When two or more types of chromophore decomposing agents are used in combination as described above, the chromophore decomposition effect may be more effective.

The chromophore to be decomposed in this step may be, for example, an organic dye and/or an inorganic pigment. Specifically, the chromophore to be decomposed may comprise at least one selected from the group consisting of quinone-based dyes, azo-based dyes, and inorganic pigments.

More specifically, the chromophore may be at least one selected from the group consisting of alizarin, purpurin, munjistin, laccaic, Disperse Blue 79, Disperse Blue 167, Disperse Blue 183, Red 2G, Acid Orange 5, Direct Blue, methyl orange, methyl red, and methyl yellow.

### Adsorption with an ion-exchange resin

Next, the chromophore in the decomposition product is adsorbed and removed with an ion-exchange resin to obtain a purified product.

As it is subjected to the ion exchange, the chromophore and by-products present in the decomposition product may be removed.

As is well known, an ion-exchange resin refers to a resin or polymer that serves as a medium for ion exchange. The ion-exchange resin may comprise a cation-exchange resin, an anion-exchange resin, an amphoteric ion-exchange resin, a chelate resin, or the like.

The cation-exchange resin may comprise a strongly acidic cation-exchange resin having a sulfonic acid group (-SO₃H) and a weakly acidic cation-exchange resin having a carboxyl group (-COOH). The anion-exchange resin may comprise a strongly basic anion-exchange resin in the form of a quaternary ammonium salt and a weakly basic anion-exchange resin having a primary to tertiary amino group.

In a specific example, the adsorption with an ion-exchange resin may comprise an adsorption step using a strongly acidic cation-exchange resin; and an adsorption step using a strongly basic anion-exchange resin. When the above two steps are performed, the efficiency of removing ionic substances is significantly enhanced as compared with the case where only one adsorption step using a cation- or anion-exchange resin is performed, whereby it is possible to improve the yellow index and color of a final product.

According to an embodiment, the ion exchange is carried out by adding an ion-exchange resin to the decomposition product (reaction result of the depolymerization product with the chromophore decomposing agent).

The weight of the ion-exchange resin added may be 1, 3, or 5 times the weight of the catalyst added in the depolymerization reaction or more, and may be 20, 15, 10, or 8 times or less. For example, the weight of the ion-exchange resin added may be 1 to 20 times, specifically, 3 to 15 times, more specifically, 5 to 8 times, relative to the weight of the catalyst added in the depolymerization reaction.

In addition, the weight of the ion-exchange resin added may be 1 part by weight or more, 3 parts by weight or more, or 5 parts by weight or more, and may be 50 parts by weight or less, 20 parts by weight or less, 15 parts by weight or less, 10 parts by weight or less, or 7 parts by weight or less, relative to 100 parts by weight of the waste polyester resin employed in the depolymerization reaction.

As a specific example, the ion-exchange resin may be used in an amount of 1 part by weight to 20 parts by weight relative to 100 parts by weight of the waste polyester.

According to another embodiment, the ion exchange is carried out by using a column containing an ion-exchange resin.

Specifically, the column may be filled with particles of an ion-exchange resin, and ion exchange may be carried out while the decomposition product (reaction result of the depolymerization product with the chromophore decomposing agent) passes through the column.

The particle diameter of the ion-exchange resin particles may be, for example, 0.3 mm to 1.5 mm, more specifically, 0.6 mm to 0.9 mm.

The temperature for ion exchange may be, for example, 140°C or lower, 130°C or lower, 120°C or lower, 110°C or lower, or 100°C or lower, and may be 50°C or higher, 60°C or higher, 70°C or higher, 80°C or higher, or 90°C or higher.

The ion-exchange resin can adsorb the chromophore contained in the decomposition product. The specific types of the chromophore are as described above.

In addition, the ion-exchange resin can adsorb at least one selected from the group consisting of halide ions, metal ions, charged fine particles, and conjugate acid or base ions thereof, in addition to the chromophore.

In addition, the ion-exchange resin can adsorb the chromophore decomposing agent added to the depolymerization product in the previous step.

### Crystallization

The purified product obtained by the adsorption with an ion-exchange resin is subjected to crystallization.

The process according to an embodiment may further comprise, before the crystallization, adding an adsorbent to the purified product and carrying out adsorption.

A carbon-based inorganic adsorbent can be used as the adsorbent. Specifically, activated carbon, activated clay, or the like may be used. The shape of the adsorbent is not particularly limited. As an example, the adsorbent may comprise activated carbon.

Sub-micron-sized particles, undissolved organic substances, and the like in the purified product can be adsorbed and removed by the adsorbent.

Adsorption by the adsorbent may be carried out in an aqueous solution. Specifically, activated carbon may be added to the purified product in the aqueous solution state.

The amount of activated carbon used may be 0.01 part by weight or more, 0.1 part by weight or more, 0.3 part by weight or more, 0.5 part by weight or more, 0.7 part by weight or more, or 1 part by weight or more, and may be 10 parts by weight or less, 5 parts by weight or less, 3 parts by weight or less, 2 parts by weight or less, or 1.5 parts by weight or less, relative to 100 parts by weight of bis(2-hydroxyethyl) terephthalate contained in the purified product.

As a specific example, the activated carbon may be used in an amount of 0.5 part by weight to 2 parts by weight relative to 100 parts by weight of bis(2-hydroxyethyl) terephthalate contained in the purified product. Within the above preferred range, the adsorption effect may be more excellent. More specifically, the activated carbon may be used in an amount of 1 part by weight to 2 parts by weight relative to 100 parts by weight of bis(2-hydroxyethyl) terephthalate contained in the purified product.

The filtrate obtained through the adsorbent may be subjected to crystallization.

Various solvents may be used for the crystallization, but a solvent capable of dissolving bis(2-hydroxyethyl) terephthalate is preferably used as a solvent. As a specific example, in order to obtain the final reactant, water as a solvent is added to the recycled bis(2-hydroxyethyl) terephthalate, which is dissolved by heating, and an adsorbent is added thereto, followed by subjecting the solution obtained by filtration to crystallization and final filtration. As a result, bis(2-hydroxyethyl) terephthalate with high purity can be obtained.

Water may be added in an amount of 100 parts by weight to 500 parts by weight, specifically, 200 parts by weight to 400 parts by weight, more specifically, 300 parts by weight to 350 parts by weight, relative to 100 parts by weight of the recycled bis(2-hydroxyethyl) terephthalate.

In addition, the dissolution temperature may be 50°C to 95°C, specifically, 60°C to 85°C, more specifically, 70°C to 75°C.

The initial temperature of the crystallization reaction may be, for example, 40°C or higher, 50°C or higher, or 60°C or higher, and may be 90°C or lower, 80°C or lower, or 70°C or lower. In addition, the termination temperature of the crystallization reaction may be, for example, 0°C or higher, 10°C or higher, 15°C or higher, or 20°C or higher, and may be 35°C or lower, 30°C or lower, or 25°C or lower. The crystallization reaction may be carried out with stepwise cooling.

In an embodiment, the crystallization is carried out in an aqueous solution. It may be carried out by gradually cooling the aqueous solution at 40°C to 70°C to 15°C to 35°C.

### Recycled bis(2-hydroxyethyl) terephthalate

The present invention provides recycled bis(2-hydroxyethyl) terephthalate obtained by the process described above.

In the present specification, bis(2-hydroxyethyl) terephthalate (BHET) obtained by the depolymerization of waste polyester as described above is referred to as "recycled bis(2-hydroxyethyl) terephthalate (recycled BHET)," or abbreviated as r-BHET or rBHET, which needs to be understood as distinct from a pure BHET compound.

Meanwhile, recycled bis(2-hydroxyethyl) terephthalate may contain reagents or solvents used in various chemical steps during the depolymerization of waste polyester, or by-products formed by side reactions with them. These impurities may remain in trace amounts even after several rounds of purification. Thus, recycled BHET generally contains trace amounts of organic and inorganic impurities in addition to BHET as the main component. For this reason, recycled BHET can also be viewed as a kind of composition comprising two or more components, i.e., a BHET composition. Such recycled BHET may be used as a polymerization raw material for the preparation of a polyester resin.

Recycled bis(2-hydroxyethyl) terephthalate according to the present invention has excellent color and quality although it is obtained by the depolymerization of waste polyester.

In an embodiment, the recycled bis(2-hydroxyethyl) terephthalate may have a yellow index (YI) of 7 or less when measured for a solution dissolved in ethylene glycol at a concentration of 50% by weight. Specifically, the yellow index may be 7 or less, 6 or less, 5 or less, 4.5 or less, 4 or less, 3.5 or less, 3 or less, 2.5 or less, 2 or less, 1.5 or less, or 1 or less. As a specific example, the yellow index may be 0 to 7, 0 to 6, 0 to 5, 0 to 4, 0 to 3, 1 to 7, 1 to 6, 1 to 5, 1 to 4, 1 to 3, 2 to 7, 2 to 6, 2 to 5, 2 to 4, or 0 to 3.

For example, the yellow index may be measured for a solution obtained by dissolving recycled bis(2-hydroxyethyl) terephthalate and ethylene glycol at a weight ratio of 1:1 in an oven at 170°C and thermally treating it for 1 hour using a colorimeter at an observer angle of 2° with Illuminant D65.

The purity of the recycled BHET may be measured using liquid chromatography or the like. Specifically, the purity of the recycled BHET may be calculated by measuring the fraction (%) of the peak area of BHET out of the total peak area in a spectrum obtained using high-performance liquid chromatography (HPLC).

For example, the purity of the recycled BHET may be 95% or more, 97% or more, 98% or more, 99% or more, or 99.5% or more, and specifically 95% to 100% or 97% to 100%.

The recycled bis(2-hydroxyethyl) terephthalate according to an embodiment may have a peak area fraction of bis(2-hydroxyethyl) terephthalate of 97% or more, more specifically, 98% or more, 99% or more, or 99.5% or more, when measured by high-performance liquid chromatography (HPLC).

Meanwhile, the recycled bis(2-hydroxyethyl) terephthalate may comprise a compound other than BHET, specifically, BHET analogues, BHET oligomers (e.g., dimers, trimers), and esters (e.g., DEG esters).

In addition, the recycled bis(2-hydroxyethyl) terephthalate may have a peak area fraction of monohydroxyethyl terephthalate (MHET) of 5% or less, 3% or less, 2% or less, 1.5% or less, 1% or less, or 0.5% or less, and 0% or more or 0.001% or more, when measured by high-performance liquid chromatography (HPLC). As a specific example, the recycled bis(2-hydroxyethyl) terephthalate may have a peak area fraction of monohydroxyethyl terephthalate (MHET) of 2% or less, specifically, 0.001% to 2%, when measured by high-performance liquid chromatography (HPLC).

In addition, the recycled bis(2-hydroxyethyl) terephthalate may have a peak area fraction of BHET oligomers of dimers or higher, in total, of 5% or less, 3% or less, 2% or less, 1.5% or less, 1% or less, or 0.5% or less, and 0% or more or 0.001% or more, when measured by high-performance liquid chromatography (HPLC). As a specific example, the recycled bis(2-hydroxyethyl) terephthalate may have a peak area fraction of BHET oligomers of dimers or higher, in total, of 1% or less, specifically, 0.001% to 1%, when measured by high-performance liquid chromatography (HPLC).

In addition, the recycled bis(2-hydroxyethyl) terephthalate may have a peak area fraction of diethylene glycol (DEG) esters, in total, of 2% or less, 1.5% or less, 1% or less, 0.7% or less, 0.6% or less, or 0.5% or less, and 0% or more or 0.001% or more, when measured by high-performance liquid chromatography (HPLC). As a specific example, the recycled bis(2-hydroxyethyl) terephthalate may have a peak area fraction of diethylene glycol (DEG) esters, in total, of 0.5% or less, specifically, 0.001% to 0.5%, when measured by high-performance liquid chromatography (HPLC). The diethylene glycol ester compound may comprise 2-hydroxyethyl[2-(2-hydroxyethoxy)ethyl] terephthalate and bis[2-(2-hydroxyethoxy)ethyl]benzene-1,4-dicarboxylate.

### Preparation of a polyester resin

According to another aspect of the present invention, there is provided a process for preparing a polyester resin, which comprises (1) obtaining a depolymerization product by glycolysis of waste polyester; (2) reacting the depolymerization product with a chromophore decomposing agent to obtain a decomposition product in which a chromophore is decomposed; (3) adsorbing and removing the chromophore in the decomposition product with an ion-exchange resin to obtain a purified product; (4) crystallizing the purified product to obtain recycled bis(2-hydroxyethyl) terephthalate; and polymerizing a polyester resin using the recycled bis(2-hydroxyethyl) terephthalate.

In the process for preparing a polyester resin, the specific process conditions of steps (1) to (4) may be adopted as those previously described in the process for preparing recycled bis(2-hydroxyethyl) terephthalate.

Recycled bis(2-hydroxyethyl) terephthalate thus prepared is used to polymerize a polyester resin.

The polymerization of a polyester resin may comprise carrying out a polycondensation reaction (first polycondensation reaction) under a low vacuum to prepare an oligomer of a low molecular weight; and subjecting the oligomer to a polycondensation reaction (second polycondensation reaction) under a high vacuum to prepare a polyester resin.

The first polycondensation reaction and the second polycondensation reaction may be carried out under a reduced pressure condition to discharge the solvent contained in the recycled bis(2-hydroxyethyl) terephthalate and by-products (glycols or the like) of the polycondensation reaction to the outside of the system.

The pressure during the first polycondensation reaction may be, for example, 700 mmHg or less, 600 mmHg or less, 500 mmHg or less, 400 mmHg or less, 350 mmHg or less, 300 mmHg or less, or 250 mmHg or less, and may be 160 mmHg or more, 180 mmHg or more, 200 mmHg or more, 220 mmHg or more, or 240 mmHg or more. According to an embodiment, the pressure during the first polycondensation reaction is 200 mmHg to 600 mmHg. Within the above preferred range, it may be more advantageous for sufficiently removing by-products of the polycondensation reaction under a low vacuum while maintaining the degree of vacuum during the polycondensation reaction.

In addition, the temperature during the first polycondensation reaction may be, for example, 100°C or higher, 130°C or higher, 160°C or higher, 180°C or higher, or 200°C or higher, and may be 300°C or lower, 280°C or lower, 250°C or lower, or 230°C or lower. As a specific example, the first polycondensation reaction may be carried out at a temperature of 180°C to 250°C and a pressure of 200 mmHg to 400 mmHg.

In addition, the first polycondensation reaction may be carried out until the number average molecular weight of the oligomer of a low molecular weight reaches an appropriate level. The period of time required for the first polycondensation reaction is not particularly limited, but it may be, for example, 30 minutes or more, 1 hour or more, 2 hours or more, or 3 hours or more, and may be 15 hours or less, 10 hours or less, 5 hours or less, or 4 hours or less. Specifically, it may be 1 hour to 5 hours.

The pressure during the second polycondensation reaction may be, for example, less than 200 mmHg, 150 mmHg or less, 100 mmHg or less, 50 mmHg or less, 10 mmHg or less, or 1 mmHg or less, and may be 0.001 mmHg or more, 0.01 mmHg or more, 0.1 mmHg or more, or 0.5 mmHg or more. According to an embodiment, the pressure during the second polycondensation reaction is less than 200 mmHg. Within the above preferred range, it may be more advantageous for sufficiently removing by-products of the polycondensation reaction while maintaining the degree of vacuum during the polycondensation reaction.

In addition, the temperature during the second polycondensation reaction may be, for example, 230°C or higher, 240°C or higher, 250°C or higher, or 260°C or higher, and may be 300°C or lower, 290°C or lower, 280°C or lower, or 270°C or lower. As a specific example, the second polycondensation reaction may be carried out at a temperature of 250°C to 300°C and a pressure of 0.01 mmHg to 150 mmHg. Within the above preferred range, it may be more advantageous for sufficiently removing by-products of the polycondensation reaction, thereby suppressing the yellowing of a final resin, while maintaining the degree of vacuum during the polycondensation reaction.

In addition, the second polycondensation reaction may be carried out until the number average molecular weight of the polyester resin reaches an appropriate level. The period of time required for the second polycondensation reaction is not particularly limited, but it may be, for example, 30 minutes or more, 1 hour or more, 2 hours or more, or 5 hours or more, and may be 60 hours or less, 48 hours or less, 24 hours or less, or 15 hours or less. Specifically, it may be 1 hour to 24 hours.

The process for preparing a polyester resin of the present invention may further comprise a step commonly employed in this field in addition to the steps described above.

As an example, the process for preparing a polyester resin may further comprise molding the polyester resin to form pellets after the polycondensation reaction.

As another example, the process for preparing a polyester resin may further comprise subjecting the polyester resin to a solid-state polymerization after the polycondensation reaction. The temperature during the solid-state polymerization may be, for example, 180°C or higher, 190°C or higher, 200°C or higher, or 205°C or higher, and may be 260°C or lower, 240°C or lower, 220°C or lower, or 215°C or lower. As a specific example, the solid-state polymerization may be carried out at a temperature of 200°C to 220°C. In addition, the pressure during the solid-state polymerization may be, for example, 10.0 Torr or less, 5.0 Torr or less, 2.0 Torr or less, or 1.0 Torr or less, and may be 0.01 Torr or more, 0.1 Torr or more, 0.2 Torr or more, or 0.5 Torr or more. Specifically, it may be 0.2 Torr to 2.0 Torr. In addition, the solid-state polymerization may be carried out in an inert gas atmosphere such as nitrogen.

The polyester resin according to the present invention may be prepared as a copolymerized polyester resin by further adding an additional diacid component in addition to the recycled bis(2-hydroxyethyl) terephthalate. The additional diacid component may be a dicarboxylic acid or a derivative thereof. The dicarboxylic acid may comprise at least one selected from terephthalic acid and isophthalic acid. For example, a dicarboxylic acid or a derivative thereof may be additionally added during the first polycondensation reaction.

In addition, the polycondensation reaction may be carried out in the presence of a polycondensation catalyst. The polycondensation catalyst may be selected for use from a group consisting of a titanium-based compound, a germanium-based compound, an antimony-based compound, and an aluminum-based compound. The amount of the polycondensation catalyst used is preferably 0.1 ppm to 500 ppm based on the amount of metal elements relative to the weight of a final polyester resin. Since the amount used has an impact on the color of the final polyester resin, the amount used may vary depending on the desired color and the stabilizer and colorant used.

In addition to the polycondensation catalyst, a stabilizer, a colorant, a crystallizing agent, an antioxidant, a branching agent, or the like may be further used. The timing of adding these additives is not particularly limited, and they may be added at any time during the preparation step of the polyester resin.

As the stabilizer, phosphorus-based compounds such as phosphoric acid, trimethyl phosphate, triethyl phosphate, and triethyl phosphonoacetate may be generally used. The amount thereof added may be such that 10 to 200 ppm relative to the weight of the polyester resin based on the amount of elements. In addition, common colorants such as cobalt acetate and cobalt propionate may be exemplified as the colorant added to enhance the color of the polyester resin. The amount thereof added may be such that 10 to 200 ppm relative to the weight of the polyester resin based on the amount of cobalt element. If necessary, anthraquinone-based compounds, perinone-based compounds, azo-based compounds, methine-based compounds, or the like may be used as an organic colorant. Commercially available toners such as Polysynthren Blue RLS from Clarient or Solvaperm Red BB from Clarient may be used. The amount of the organic compound colorant added may be adjusted to 0 to 50 ppm based on the weight of the polyester resin. A crystal nucleating agent, an ultraviolet absorber, a polyolefin resin, a polyamide resin, and the like may be exemplified as the crystallizing agent. Hindered phenol-based antioxidants, phosphite-based antioxidants, thioether-based antioxidants, or mixtures thereof may be exemplified as the antioxidant. Conventional branching agents having three or more functional groups, for example, trimellitic anhydride, trimethylol propane, trimellitic acid, or mixtures thereof may be exemplified as the branching agent.

### Composition and characteristics of the polyester resin

According to another aspect of the present invention, there is provided a polyester resin prepared by the above process.

The polyester resin thus prepared is a polyester resin regenerated through the chemical recycling of waste polyester.

Specifically, since the polyester resin of the present invention is polymerized using recycled BHET, it comprises a repeat unit derived from recycled BHET in the polymer chain.

The content of recycled BHET in the polyester resin of the present invention may be 90% by weight or more or 95% by weight or more. In addition, the content of recycled BHET may be 100% by weight or less, 99% by weight or less, or 95% by weight or less.

As an example, the recycled bis(2-hydroxyethyl) terephthalate may be employed in an amount of 90% by weight to 100% by weight based on the weight of the polyester resin.

Meanwhile, since bis(2-hydroxyethyl) terephthalate has a structure in which two ethylene glycol molecules and one terephthalic acid molecule are bonded, the polyester resin of the present invention may essentially comprise a repeat unit derived from ethylene glycol and terephthalic acid.

In addition, the polyester resin of the present invention may be a copolymerized polyester resin. For example, it may further comprise an additional diacid component as a copolymerization monomer. The additional diacid component may be a dicarboxylic acid or a derivative thereof. The dicarboxylic acid may comprise at least one selected from terephthalic acid and isophthalic acid. They may enhance the physical properties such as thermal resistance, chemical resistance, and weatherability of a polyester resin.

As described above, as the polyester resin of the present invention comprises recycled bis(2-hydroxyethyl) terephthalate of high purity and high quality, it has low impurities and excellent thermal resistance even though it is a regenerated resin.

Accordingly, the present invention provides a polyester resin prepared by the process described above. That is, in the polyester resin of the present invention, the peak area fraction of bis(2-hydroxyethyl) terephthalate is 96% or more, and the peak area fraction of diethylene glycol (DEG) ester compounds is less than 2% in total, when measured by high-performance liquid chromatography (HPLC).

In addition, the polyester resin may have, for example, a concentration of diethylene glycol of 2.5% by weight or less, 1.5% by weight or less, 1.2% by weight or less, 1.0% by weight or less, or 0.9% by weight or less, when measured by gas chromatography. As a specific example, the polyester resin may have a concentration of diethylene glycol of 0.8% by weight or less when measured by gas chromatography.

The intrinsic viscosity of the polyester resin of the present invention at 35°C may be, for example, 0.5 dl/g or more, 0.6 dl/g or more, 0.7 dl/g or more, 0.75 dl/g or more, 0.76 dl/g or more, or 0.8 dl/g or more, and may be 1.2 dl/g or less, 1.1 dl/g or less, 1.0 dl/g or less, or 0.9 dl/g or less. According to an embodiment, the polyester resin may have an intrinsic viscosity at 35°C of 0.6 dl/g to 1.2 dl/g. The intrinsic viscosity may be calculated, for example, by dissolving the polyester resin in a solvent such as orthochlorophenol and obtaining specific viscosity using an Ubbelohde viscometer or the like.

In addition, the polyester resin of the present invention may have a glass transition temperature (Tg) of, for example, 65°C or higher, 70°C or higher, 75°C or higher, or 79.5°C or higher, and may be 100°C or lower, 95°C or lower, 90°C or lower, or 85°C or lower. As a specific example, the glass transition temperature (Tg) of the polyester resin may be 75°C to 95°C or 79.5°C to 90°C. The melting point may be measured by, for example, a method comprising putting the polyester resin in a differential scanning calorimeter (DSC) and raising the temperature from room temperature to 280°C at a constant rate.

In addition, the polyester resin of the present invention may have a melting point (Tm) of, for example, 240°C or higher, 250°C or higher, 254.5°C or higher, or 255°C or higher, and may be 290°C or lower, 280°C or lower, 270°C or lower, or 260°C or lower. As a specific example, the melting point (Tm) of the polyester resin may be 240°C to 290°C or 254.5°C to 280°C.

The glass transition temperature (Tg) and melting point (Tm) of the polyester resin may be measured from a heat flow obtained when a sample is filled in an aluminum pan, heated up to 280°C at 10°C/minute, maintained at 280°C for 5 minutes, and then cooled down to 30°C at -300°C/minute; and, subsequently, the temperature was raised to 280°C at 10°C/minute, using differential scanning calorimetry (DSC).

In addition, the polyester resin may have an L* value of 60 or more, 70 or more, 80 or more, 85 or more, 86 or more, or 87 or more, and 90 or less, when CIE Lab color space is measured. As a specific example, the polyester resin may have an L* value of 60 to 90, 70 to 90, 80 to 90, 86 to 90, or 87 to 90, when CIE Lab color space is measured.

In addition, the polyester resin may have a b* value of 10 or less, 8 or less, 6 or less, 5 or less, 4 or less, 3 or less, 2 or less, or 1 or less, and 0 or more, when CIE Lab color space is measured. As a specific example, the polyester resin may have a b* value of 0 to 10, 0 to 6, 0 to 5, 0 to 4, or 0 to 3, when CIE Lab color space is measured.

The polyester resin may have a value of (L* - b*) obtained by subtracting the b* value from the L* value of 50 or more, 60 or more, 70 or more, 80 or more, 86 or more, or 87 or more, when Hunter Lab color space is measured. In addition, the upper limit of the (L* - b*) value is not particularly limited. But it may be, for example, 100 or less, 97 or less, 95 or less, 93 or less, or 90 or less. As a specific example, the (L* - b*) value may be 70 to 100, 80 to 100, 70 to 90, or 80 to 90.

### Mode for the Invention

Hereinafter, a preferred embodiment is presented for the understanding of the present invention. However, the following examples are provided only to help easily understand the present invention, and the scope of the present invention is not limited thereby.

### <Preparation example of recycled bis(2-hydroxyethyl) terephthalate>

### Example A1

A first reactor made of stainless steel (SUS) was charged with 1,000 g of a waste polyester resin, 2,000 g of ethylene glycol, and 5.0 g of zinc acetate anhydride. The temperature inside the reactor was raised to 180°C, and depolymerization (first glycolysis reaction) was carried out for 2 hours. The reactant thus obtained was transferred to a second reactor and cooled to 150°C. 2,000 g of ethylene glycol was further added thereto, and depolymerization (second glycolysis reaction) was carried out for 2 hours while the reactor temperature was maintained at 150°C.

The reactant thus obtained was cooled to 110°C, followed by pressurized filtration to carry out solid-liquid separation. The separated liquid reactant was passed through a column filled with an ion-exchange resin (BC107(H) of Bonlite) to remove ionic impurities to obtain a mixture containing bis(2-hydroxyethyl) terephthalate and ethylene glycol. The mixture was transferred to a 10-liter distillation apparatus, and vacuum distillation was carried out at 130°C to recover unreacted ethylene glycol. The reactant from which ethylene glycol had been removed was subjected to thin film evaporation at 220°C and 0.08 Torr in a thin film evaporator (VKL70-4S of VTA) to obtain 1,040 g of a product from which dimers or higher oligomers had been removed. Thereafter, the obtained result and 3,120 g of distilled water were mixed in a 20-liter glass reactor and heated to 70°C to obtain a crude aqueous BHET solution.

2.6 g of chloramine-T (CAT) and 2.6 g of NaClO were added to the crude aqueous BHET solution, and a chromophore decomposition reaction was carried out for 1 hour. The obtained decomposition product (the result of the decomposition reaction) was sequentially passed through a column filled with a cation-exchange resin (BC107(H) from Bonlite) and an anion-exchange resin (TRILITE^{®} SAR10MBOH from Samyang) to obtain a purified solution. 5.2 g of activated carbon was additionally added to the purified solution, which was stirred for 30 minutes and then filtered. The filtrate was cooled to room temperature for the crystallization thereof, filtered, and dried in a vacuum oven. As a result, recycled bis(2-hydroxyethyl) terephthalate with excellent color and purity was obtained.

### Example A2

The same procedure as in Example A1 was repeated to obtain recycled bis(2-hydroxyethyl) terephthalate, except that the amount of chloramine-T was changed to 1.3 g.

### Example A3

The same procedure as in Example A1 was repeated to obtain recycled bis(2-hydroxyethyl) terephthalate, except that HClO₄ instead of NaClO was used in the same amount.

### Example A4

The same procedure as in Example A1 was repeated to obtain recycled bis(2-hydroxyethyl) terephthalate, except that an H₂O₂ solution instead of NaClO was used in the same amount.

### Example A5

The same procedure as in Example A1 was repeated to obtain recycled bis(2-hydroxyethyl) terephthalate, except that NaClO was not used.

### Example A6

The same procedure as in Example A1 was repeated to obtain recycled bis(2-hydroxyethyl) terephthalate, except that the amount of activated carbon was changed to 10.4 g.

### Comparative Example A1

The same procedure as in Example A1 was repeated to obtain recycled bis(2-hydroxyethyl) terephthalate, except that none of chloramine-T and NaClO were used.

### Comparative Example A2

The same procedure as in Example A1 was repeated to obtain recycled bis(2-hydroxyethyl) terephthalate, except that none of chloramine-T and NaClO were used and that the amount of activated carbon was changed to 10.4 g.

### Comparative Example A4

The same procedure as in Example A1 was repeated to obtain recycled bis(2-hydroxyethyl) terephthalate, except that, after the chromophore removal reaction, it was not passed through a column filled with a cation-exchange resin (BC107(H) from Bonlite) and an anion-exchange resin (TRILITE^{®} SAR10MBOH from Samyang).

The processes for preparing recycled bis(2-hydroxyethyl) terephthalate of the Examples and Comparative Examples were summarized in the tables below.

**[Table 1]**

| | Info. | Ex. A1 | Ex. A2 | Ex. A3 | Ex. A4 | Ex. A5 | Ex. A6 |
|---|---|---|---|---|---|---|---|
| | Decomposing agent 1 | CAT | CAT | CAT | CAT | CAT | CAT |
| rBHET process | Decomposing agent 2 | NaClO | NaClO | HClO₄ | H₂O₂ | - | - |
| | Ion exchange | Cation/anion | Cation/anion | Cation/anion | Cation/anion | Cation/anion | Cation/anion |
| | Adsorbent | Activated carbon | Activated carbon | Activated carbon | Activated carbon | Activated carbon | Activated carbon |

**[Table 2]**

| | Info | Comp. Ex. A1 | Comp. Ex. A2 | Comp. Ex. A4 |
|---|---|---|---|---|
| | Decomposing agent 1 | - | - | CAT |
| rBHET process | Decomposing agent 2 | - | - | NaClO |
| | Ion exchange | Cation/anion | Cation/anion | - |
| | Adsorbent | Activated carbon | Activated carbon | Activated carbon |

### <Preparation example of a recycled polyester resin>

### Example B1

990 g of recycled bis(2-hydroxyethyl) terephthalate (r-BHET) prepared in Example A1, 156 g of water, and 242 g of ethylene glycol (EG) were homogeneously mixed at 70°C to prepare an r-BHET solution (concentration: 71.4% by weight). A 7-liter reactor capable of a reaction under vacuum was charged with 1,388 g of the solution of recycled bis(2-hydroxyethyl) terephthalate (r-BHET) prepared above, 0.4 g of antimony trioxide as a catalyst, 0.3 g of triethyl phosphate as a stabilizer, and 0.2 g of cobalt acetate as a coloring agent. The temperature of the reactor was raised to 190°C over 2 hours. When the temperature reached 190°C, the pressure of the reactor was reduced from normal pressure to 200 Torr (absolute pressure: 200 mmHg) over 30 minutes. A polycondensation reaction under a low vacuum was carried out for 1 hour while the pressure of the reactor was maintained at 200 Torr (absolute pressure: 200 mmHg). The pressure of the reactor was reduced from 200 Torr (absolute pressure: 200 mmHg) to 5 Torr (absolute pressure: 5 mmHg) over 30 minutes. At the same time, the temperature of the reactor was raised to 280°C over 1 hour, and a polycondensation reaction under a high vacuum was carried out while the pressure of the reactor was maintained at 1 Torr (absolute pressure: 1 mmHg) or less. The mixture in the reactor was discharged to the outside of the reactor to form strands, which were solidified with a cooling liquid and then granulated to have an average weight of about 12 to 14 mg. The granules were left at 150°C for 1 hour to be crystallized, which were then fed to a 20-liter reactor for a solid-state polymerization. Thereafter, nitrogen flowed into the reactor at a rate of 50 liters/minute. In such an event, the temperature of the reactor was raised from room temperature to 140°C at a rate of 40°C/hour and maintained at 140°C for 3 hours to obtain a recycled polyester resin.

### Example B2

The same procedure as in Example B1 was repeated to obtain a recycled polyester resin, except that the recycled bis(2-hydroxyethyl) terephthalate (r-BHET) prepared in Example A2 was used.

### Example B3

The same procedure as in Example B1 was repeated to obtain a recycled polyester resin, except that the recycled bis(2-hydroxyethyl) terephthalate (r-BHET) prepared in Example A3 was used.

### Example B4

The same procedure as in Example B1 was repeated to obtain a recycled polyester resin, except that the recycled bis(2-hydroxyethyl) terephthalate (r-BHET) prepared in Example A4 was used.

### Example B5

The same procedure as in Example B1 was repeated to obtain a recycled polyester resin, except that the recycled bis(2-hydroxyethyl) terephthalate (r-BHET) prepared in Example A5 was used.

### Example B6

The same procedure as in Example B1 was repeated to obtain a recycled polyester resin, except that the recycled bis(2-hydroxyethyl) terephthalate (r-BHET) prepared in Example A6 was used.

### Comparative Example B1

The same procedure as in Example B1 was repeated to obtain a recycled polyester resin, except that the recycled bis(2-hydroxyethyl) terephthalate (r-BHET) prepared in Comparative Example A1 was used.

### Comparative Example B2

The same procedure as in Example B1 was repeated to obtain a recycled polyester resin, except that the recycled bis(2-hydroxyethyl) terephthalate (r-BHET) prepared in Comparative Example A2 was used.

### Comparative Example B4

The same procedure as in Example B1 was repeated to obtain a recycled polyester resin, except that the recycled bis(2-hydroxyethyl) terephthalate (r-BHET) prepared in Comparative Example A4 was used.

### Test Example 1

The recycled bis(2-hydroxyethyl) terephthalate prepared in each of the Examples and Comparative Examples was tested as follows.

### (1) HPLC

About 0.01 g of recycled BHET was diluted in about 20 ml of methanol, which was analyzed by high-performance liquid chromatography (HPLC) (model: Waters e2695, column: C18 (4.6 × 250 mm), 5 µm, UV detector: 242 nm, injection volume: 10 µl, eluent (gradient) A: H₂O + H₃PO₄, B: acetonitrile). Thereafter, the peak area fractions (%) of the following components among the total peak area of HPLC were obtained.
- MHET: monohydroxyethyl terephthalate
- BHET: bis(2-hydroxyethyl) terephthalate,
- DEG-ester-1: 2-hydroxyethyl[2-(2-hydroxyethoxy)ethyl] terephthalate
- DEG-ester-2: bis[2-(2-hydroxyethoxy)ethyl]benzene-1,4-dicarboxylate
- Dimer/trimer: BHET dimer/trimer

### (2) Absorbance

Each crude aqueous BHET solution (after thin film evaporation) subjected to the chromophore decomposition reaction in the Examples and Comparative Examples was measured for the UV-Vis absorbance using an Agilent model (Cary 4000) together with a reference sample in a transmission mode. The absorbance (arbitrary units) at a wavelength of 400 nm is shown in Table 3 below. In addition, the absorbance spectra of some Examples are shown in Fig. 1.

### (3) Yellow index (accelerated YI measurement)

20 g of ethylene glycol and 20 g of recycled BHET were mixed, dissolved in an oven at 170°C, and thermally treated for 1 hour. Transmission data for the solution were obtained with Illuminant D65 using Color Flex EZ of Hunterlab at an observer's angle of 2°. The yellow index (YI) value was calculated using a color analyzer in the software.

The results are shown in the tables below.

**[Table 3]**

| | | Info. | Ex. A1 | Ex. A2 | Ex. A3 | Ex. A4 | Ex. A5 | Ex. A6 |
|---|---|---|---|---|---|---|---|---|
| | HPLC | MHET | 1.78 | 0.89 | 3.12 | 1.32 | 1.02 | 1.05 |
| | | BHET | 97.20 | 98.10 | 96.01 | 97.44 | 97.64 | 97.58 |
| | | DEG-ester-1 | 0.41 | 0.41 | 0.37 | 0.52 | 0.46 | 0.43 |
| r-BHET compo'n | | DEG-ester-2 | 0.04 | 0.04 | 0.02 | 0.04 | 0.05 | 0.03 |
| | | Dimer | 0.21 | 0.35 | 0.29 | 0.35 | 0.65 | 0.54 |
| | | Trimer | 0.01 | 0.01 | 0.01 | 0.01 | 0.01 | 0.01 |
| | | Others | 0.35 | 0.20 | 0.18 | 0.32 | 0.17 | 0.36 |
| | Absorbance | at 400 nm | 0.056 | 0.079 | 0.067 | 0.066 | 0.086 | 0.063 |
| | Color | YI | 2.30 | 4.80 | 3.90 | 4.20 | 6.60 | 4.50 |

**[Table 4]**

| | | Info. | Comp. Ex. A1 | Comp. Ex. A2 | Comp. Ex. A4 |
|---|---|---|---|---|---|
| | HPLC | MHET | 0.89 | 1.81 | 2.55 |
| | | BHET | 98.10 | 96.88 | 96.43 |
| | | DEG-ester-1 | 0.41 | 0.56 | 0.65 |
| r-BHET compositi on | | DEG-ester-2 | 0.04 | 0.04 | 0.04 |
| | | Dimer | 0.35 | 0.34 | 0.22 |
| | | Trimer | 0.01 | 0.02 | 0.01 |
| | | Others | 0.20 | 0.35 | 0.10 |
| | Absorbance | at 400 nm | 0.211 | 0.164 | 0.116 |
| | Color | YI | 61.00 | 29.50 | 8.60 |

As can be seen from the above tables, the recycled BHET obtained in Examples A1 to A6 had a lower content of impurities, along with lower absorbance and yellow index, than the recycled BHET obtained in Comparative Examples A1 to A4, indicating excellent quality and color.

### Test Example 2

The polyester resins of the Examples and Comparative Examples were each tested as follows.

### (1) DSC

A differential scanning calorimeter (DSC, Q20 model, TA instrument) was used. Each sample was filled in an aluminum pan, heated up to 280°C at 10°C/minute, maintained at 280°C for 5 minutes, and then cooled down to 30°C at -300°C/minute. Subsequently, the glass transition temperature (T_{g}) and melting temperature (Tₘ) were obtained from the heat flow obtained when the temperature was raised to 280°C at 10°C/minute.

### (2) Intrinsic viscosity (IV)

Each polyester resin was dissolved at a concentration of 1.2 g/dl in orthochlorophenol (OCP) at 150°C to obtain a solution, and an Ubbelohde viscometer was used to measure intrinsic viscosity.

Specifically, the temperature of the viscosity tube was maintained at 35°C, and the time (efflux time) required for the solvent to pass between specific internal sections of the viscosity tube and the time required for the solution to pass to obtain specific viscosity, which was used to calculate intrinsic viscosity.

### (3) Color (for PET chip)

Each prepared polyester resin (PET chip) was used as a sample, and the color was measured using a colorimeter (CHROMA METER CR-410, Konica Minolta). The color was measured as L*, a*, and b* values of the CIE LAB color space. The CIE LAB color space is a color space coordinate defined by CIE (International Commission on Illumination) based on the opposite colors that humans perceive, such as yellow-blue and green-red. L* value represents brightness (0-100; 0 is black, 100 is white), a* value represents green-red (+ is red and - is green based on 0), and b* value represents yellow-blue (+ is yellow and - is blue based on 0).

The test results are shown in the tables below.

**[Table 5]**

| | | | Ex. B1 | Ex. B2 | Ex. B3 | Ex. B4 | Ex. B5 | Ex. B6 |
|---|---|---|---|---|---|---|---|---|
| | r-BHET | | Ex. Al | Ex. A2 | Ex. A3 | Ex. A4 | Ex. A5 | Ex. A6 |
| | Tg | (°C) | 80.2 | 79.9 | 80.0 | 80.1 | 80.3 | 80.0 |
| Recycled PET resin | Tm | (°C) | 256.2 | 257.1 | 256.2 | 255.3 | 256.4 | 255.4 |
| | IV | (g/dL) | 0.81 | 0.81 | 0.79 | 0.80 | 0.81 | 0.82 |
| | Color | L* | 90.0 | 88.6 | 89.4 | 89.0 | 87.9 | 89.2 |
| | | b* | 0.9 | 2.1 | 1.2 | 1.9 | 2.9 | 1.4 |
| | | L* - b* | 89.1 | 86.5 | 88.2 | 87.1 | 85.0 | 87.8 |

**[Table 6]**

| | | | Comp. Ex. B1 | Comp. Ex. B2 | Comp. Ex. B4 |
|---|---|---|---|---|---|
| | r-BHET | | Comp. Ex. A1 | Comp. Ex. A2 | Comp. Ex. A4 |
| Recycled PET resin | Tg | (°C) | 78.3 | 79.0 | 77.8 |
| | Tm | (°C) | 253.2 | 253.7 | 252.4 |
| | IV | (g/dL) | 0.75 | 0.81 | 0.73 |
| | Color | L* | 58.1 | 64.5 | 85.8 |
| | | b* | 9.1 | 8.5 | 6.8 |
| | | L* - b* | 49.0 | 56.0 | 79.0 |

As can be seen from the above table, the recycled polyester resins obtained in Examples B1 to B6 not only had excellent thermal properties as compared with the recycled polyester resins obtained in Comparative Examples B1 to B4, but also had a high white index, indicating excellent color.

In particular, based on the above test results, it is understood that the color of recycled BHET has a direct impact on the color of a final polyester resin. A small quality difference in the recycled BHET may cause a large quality difference in the final polyester resin.

## Claims

1. A process for preparing recycled bis(2-hydroxyethyl) terephthalate, which comprises:
(1) obtaining a depolymerization product by glycolysis of waste polyester;
(2) reacting the depolymerization product with a chromophore decomposing agent to obtain a decomposition product in which a chromophore is decomposed;
(3) adsorbing and removing the chromophore in the decomposition product with an ion-exchange resin to obtain a purified product; and
(4) crystallizing the purified product.

2. The process for preparing recycled bis(2-hydroxyethyl) terephthalate of claim 1, wherein the waste polyester has a yellow index (YI), when measured in a solid phase, of 10 or more.

3. The process for preparing recycled bis(2-hydroxyethyl) terephthalate of claim 1, wherein the step of obtaining the depolymerization product comprises:
obtaining a first depolymerization product by a first glycolysis reaction of the waste polyester at a temperature of 180 to 210°C; and
obtaining a second depolymerization product by a second glycolysis reaction of the first depolymerization product at a temperature of 150 to 170°C.

4. The process for preparing recycled bis(2-hydroxyethyl) terephthalate of claim 1, which further comprises, before step (2), distilling moisture and a residual solvent in the depolymerization product.

5. The process for preparing recycled bis(2-hydroxyethyl) terephthalate of claim 1, wherein the chromophore decomposing agent in step (2) comprises a chloroamine.

6. The process for preparing recycled bis(2-hydroxyethyl) terephthalate of claim 5, wherein the chromophore decomposing agent in step (2) further comprises at least one of chlorates, chloric acids, and hydrogen peroxide.

7. The process for preparing recycled bis(2-hydroxyethyl) terephthalate of claim 1, the chromophore to be decomposed in step (2) comprises at least one selected from the group consisting of quinone-based dyes, azo-based dyes, and inorganic pigments.

8. The process for preparing recycled bis(2-hydroxyethyl) terephthalate of claim 1, wherein the ion-exchange resin in step (3) adsorbs at least one selected from the group consisting of halide ions, metal ions, charged fine particles, and conjugate acid or base ions thereof, in addition to the chromophore.

9. The process for preparing recycled bis(2-hydroxyethyl) terephthalate of claim 1, wherein the adsorption in step (3) comprises an adsorption step using a strongly acidic cation-exchange resin; and an adsorption step using a strongly basic anion-exchange resin.

10. The process for preparing recycled bis(2-hydroxyethyl) terephthalate of claim 1, which further comprises, before the crystallization in step (4), adding an adsorbent to the purified product and carrying out adsorption, wherein the adsorbent comprises activated carbon.

11. The process for preparing recycled bis(2-hydroxyethyl) terephthalate of claim 10, wherein the activated carbon is used in an amount of 0.5 part by weight to 2 parts by weight relative to 100 parts by weight of bis(2-hydroxyethyl) terephthalate contained in the purified product.

12. The process for preparing recycled bis(2-hydroxyethyl) terephthalate of claim 1, wherein the crystallization is carried out in an aqueous solution, and it is carried out by gradually cooling the aqueous solution at 40°C to 70°C to 15°C to 35°C.

13. Recycled bis(2-hydroxyethyl) terephthalate, which is prepared by the process of claim 1, wherein the yellow index (YI) is 7 or less when measured for a solution dissolved in ethylene glycol at a concentration of 50% by weight.

14. A process for preparing a polyester resin, which comprises:
(1) obtaining a depolymerization product by glycolysis of waste polyester;
(2) reacting the depolymerization product with a chromophore decomposing agent to obtain a decomposition product in which a chromophore is decomposed;
(3) adsorbing and removing the chromophore in the decomposition product with an ion-exchange resin to obtain a purified product;
(4) crystallizing the purified product to obtain recycled bis(2-hydroxyethyl) terephthalate; and
(5) polymerizing a polyester resin using the recycled bis(2-hydroxyethyl) terephthalate.

15. A polyester resin, which is prepared by the process of claim 14.
